# EUROPEAN PATENT APPLICATION

(11) **EP 4 840 390 A1**
(43) Date of publication of application: **18.11.2026**
(21) Application number: 26177980.5
(22) Date of filing: 11.05.2026
(51) Int. Cl.: A61B 5/00, A61B 5/024, A61B 5/0205, G16H 40/63, G16H 50/30, A61B 5/01, A61B 5/08, A61B 5/021, A61B 5/145

(54) **MEDICAL DEVICE WITH ALARM ADJUSTMENT TOOL**

(30) Priority: 13.05.2025 US 202563804761 P
(71) Applicant: Welch Allyn, INC., Skaneateles Falls, NY 13153-0220 (US)
(72) Inventor: HAYWARD, Lauren N., Skaneateles Falls, 13153-0220 (US); COLES, Kathryn M., Skaneateles Falls, 13153-0220 (US); DRUM PAQUETTE, Hanna, Skaneateles Falls, 13153-0220 (US); KING, Catherine M., Skaneateles Falls, 13153-0220 (US); MYERS, Thomas A., Skaneateles Falls, 13153-0220 (US); QUILTY-KOVAL, Rebecca, Skaneateles Falls, 13153-0220 (US)
(74) Representative: Reddie & Grose LLP

(57) **Abstract**

A medical device for monitoring one or more physiological variables includes a processing device communicatively coupled to a display and at least one sensor, and a memory device storing executable instructions that cause the processing device to monitor measurements of a physiological variable over a predetermined time period to detect alarm conditions occurring when measurements exceed a default upper alarm limit or fall below a default lower alarm limit. When the quantity of alarm conditions detected during the predetermined time period exceeds a threshold, the processing device determines whether the measurements are stable within the predetermined time period. When the measurements are stable, the processing device executes a patient baseline tool that calculates adjusted alarm limits differing from the default alarm limits. The processing device then displays a trend visualization showing the measurements of the physiological variable relative to both the default alarm limits and the adjusted alarm limits.

## Description

Nuisance alarms in medical devices are false or non-urgent alerts that can cause unnecessary disruptions in healthcare environments. These alarms, while designed to notify healthcare providers of potential issues, often arise from settings that are too sensitive, improper configurations, or routine fluctuations that do not require immediate attention. The constant barrage of nuisance alarms can lead to alarm fatigue, where healthcare professionals may begin to overlook or ignore alarms, potentially jeopardizing patient safety. Addressing nuisance alarms is crucial in improving the efficiency and effectiveness of medical device systems, ensuring that only critical alerts grab the attention of healthcare professionals.

In general terms, the present disclosure relates to medical device alarm management. In one possible configuration, a medical device displays a trend visualization showing measurements of a physiological variable relative to default alarm limits and adjusted alarm limits calculated based on the measurements of the physiological variable. Various aspects are described in this disclosure, which include, but are not limited to, the following aspects.

One aspect relates to a medical device for monitoring one or more physiological variables, the medical device comprising: at least one processing device communicatively coupled to a display and to at least one sensor that captures measurements of the physiological variable; and a memory device storing instructions which, when executed by the at least one processing device, cause the at least one processing device to: monitor the measurements of the physiological variable over a predetermined time period to detect alarm conditions when the measurements of the physiological variable exceed a default upper alarm limit or are less than a default lower alarm limit; determine whether a quantity of the alarm conditions detected during the predetermined time period exceeds a threshold set for the predetermined time period; when the quantity of the alarm conditions exceeds the threshold set for the predetermined time period, determine whether the measurements of the physiological variable are stable within the predetermined time period; when the measurements of the physiological variable are stable within the predetermined time period, execute a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and display, via a graphical user interface on the display, a trend visualization showing the measurements of the physiological variable relative to the default alarm limits and the adjusted alarm limits.

Another aspect relates to a method of monitoring one or more physiological variables, the method comprising: monitoring measurements of a physiological variable over a predetermined time period to detect alarm conditions when the measurements of the physiological variable exceed a default upper alarm limit or are less than a default lower alarm limit; determining whether a quantity of the alarm conditions detected during the predetermined time period exceeds a threshold set for the predetermined time period; when the quantity of the alarm conditions exceeds the threshold set for the predetermined time period, determining whether the measurements of the physiological variable are stable within the predetermined time period; when the measurements of the physiological variable are stable within the predetermined time period, executing a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and displaying a trend visualization showing the measurements of the physiological variable relative to the default alarm limits and the adjusted alarm limits.

Another aspect relates to a computer program product storing instructions that, when executed by at least one processing device communicatively coupled to a display and to at least one sensor that captures measurements of a physiological variable, cause the at least one processing device to: monitor the measurements of the physiological variable over a predetermined time period to detect alarm conditions when the measurements of the physiological variable exceed a default upper alarm limit or are less than a default lower alarm limit; determine whether a quantity of the alarm conditions detected during the predetermined time period exceeds a threshold set for the predetermined time period; when the quantity of the alarm conditions exceeds the threshold set for the predetermined time period, determine whether the measurements of the physiological variable are stable within the predetermined time period; when the measurements of the physiological variable are stable within the predetermined time period, execute a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and display, via a graphical user interface on the display, a trend visualization showing the measurements of the physiological variable relative to the default alarm limits and the adjusted alarm limits.

The invention will now be further described by way of example with reference to the accompanying drawings, in which:
FIG. 1 is a front isometric view of a medical device.
FIG. 2 is a rear isometric view of the medical device of FIG. 1.
FIG. 3 is a front view of the medical device of FIG. 1.
FIG. 4 is a bottom view of the medical device of FIG. 1.
FIG. 5 schematically illustrates an example of the medical device of FIG. 1.
FIG. 6 schematically illustrates an example of a method of monitoring one or more physiological variables that can be performed by the medical device of FIG. 1.
FIG. 7 illustrates an example of a home screen user interface that can be displayed on a display of the medical device of FIG. 1 to display the measurements of the one or more physiological variables monitored in an operation of the method of FIG. 6.
FIG. 8 illustrates an example of a home screen user interface when an alarm condition is detected by the medical device of FIG. 1 during an operation of the method of FIG. 6.
FIG. 9 illustrates an example of an alarm tab user interface that is displayed on the display of the medical device in response to selection of an alarm tab in a menu of the home screen user interface of FIG. 8.
FIG. 10 illustrates an example of a configuration user interface that can be displayed on the display of the medical device of FIG. 1 to configure the patient baseline tool.

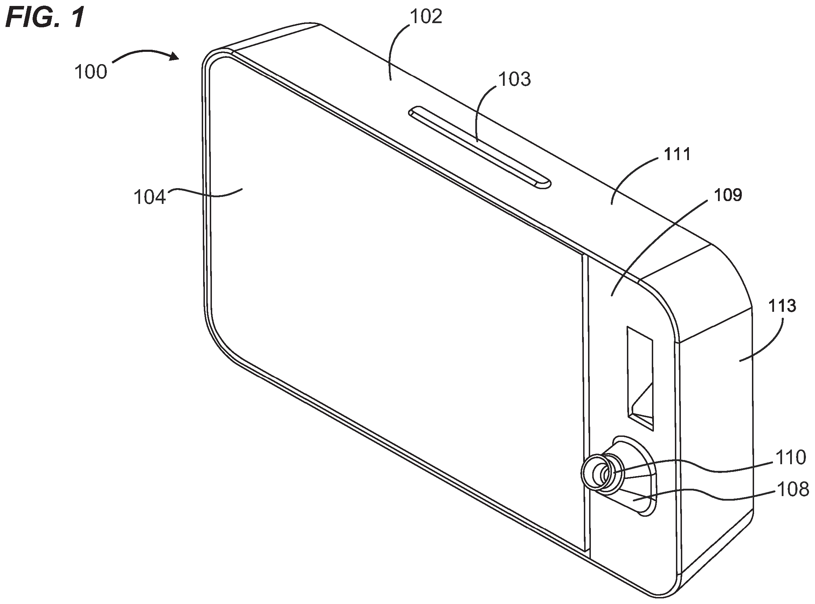

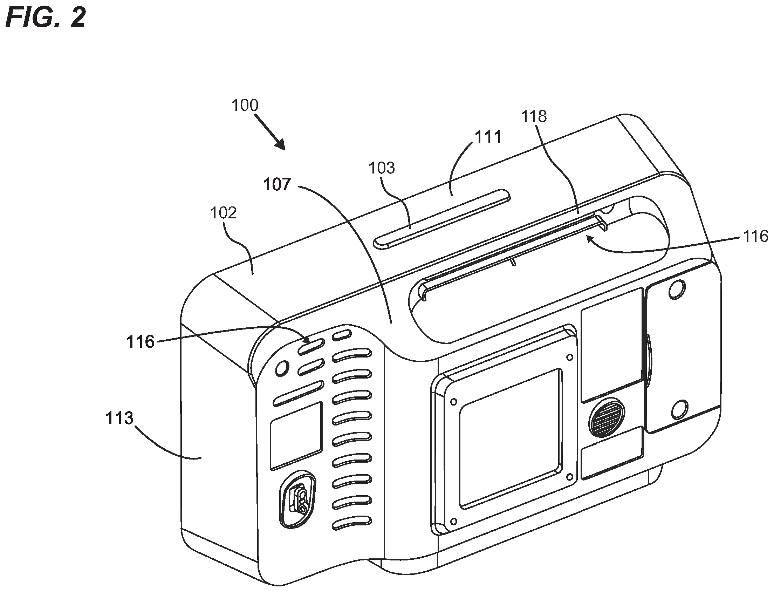

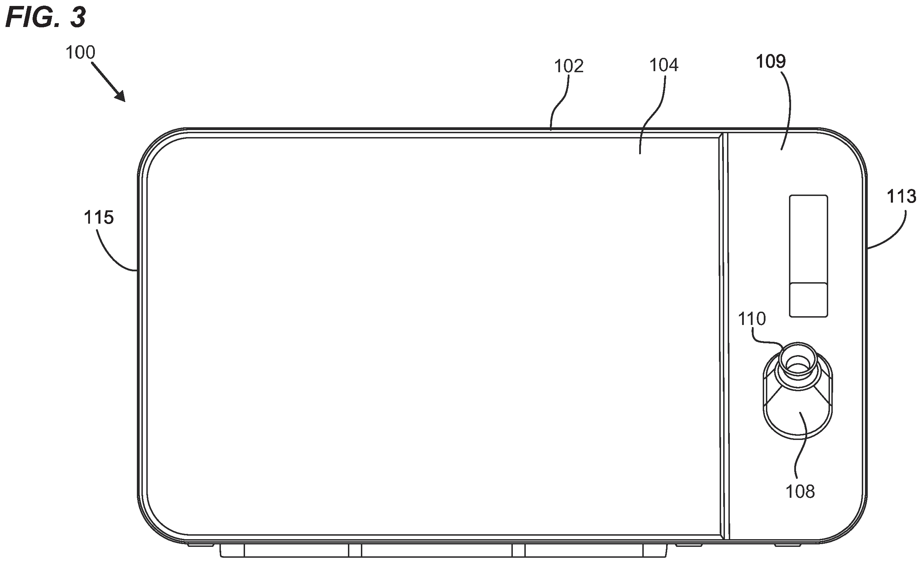

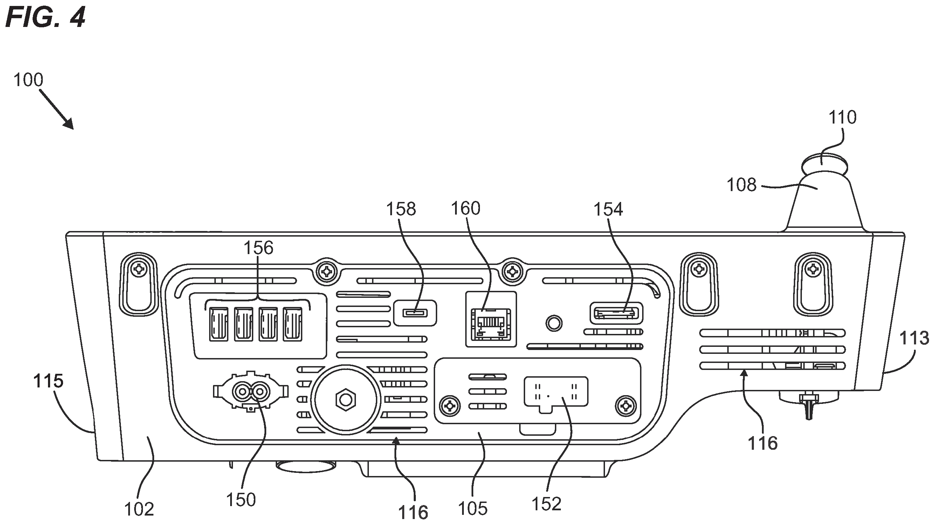

FIG. 1 is a front isometric view of a medical device 100 for monitoring one or more physiological variables of a patient. FIG. 2 is a rear isometric view of the medical device 100. FIG. 3 is a front view of the medical device 100. FIG. 4 is a bottom view of the medical device 100. As shown in FIGS. 1-4, the medical device 100 includes a housing 102 having a bottom surface 105, a rear surface 107, a front surface 109, a top surface 111, and lateral sides 113, 115 which give the medical device 100 a substantially rectangular prism shape. The industrial design shown in FIGS. 1-4 is illustrative and may vary in different examples of the medical device 100.

In some examples, the medical device 100 is portable. In such examples, the medical device 100 can include a handle 118 (see FIG. 2) that allows the housing 102 to be gripped with one hand for physically moving the medical device 100 from one location to another.

A display 104 is mounted on a front surface 109 of the housing 102. In some examples, the display 104 is a touchscreen that operates to both display data captured by one or more sensors communicatively coupled to the medical device 100 and to receive tactile inputs from a user such as to adjust one or more alarm parameters and/or to silence an alarm. The housing 102 can also support physical push buttons to receive tactile inputs from the user.

The medical device 100 is designed for use in a clinical setting. For example, the medical device 100 can be used to continuously monitor one or more physiological variables of a patient admitted to a medical facility such as a hospital, a medical clinic, and the like. Also, the medical device 100 can operate as a spot monitor for interval or episodic monitoring of one or more physiological variables of a plurality of patients in the medical facility.

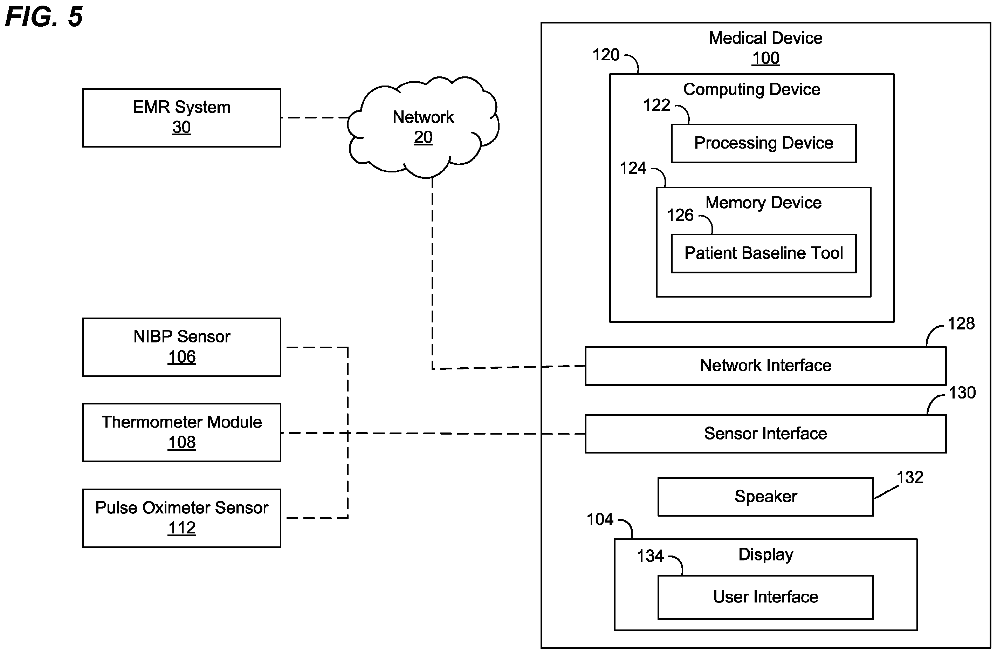

The medical device 100 is configured for connection to one or more sensors (see FIG. 5) that can be used to measure one or more physiological variables including, without limitation, non-invasive systolic and diastolic blood pressure, blood oxygen saturation (SpO2), respiration rate, pulse rate, and body temperature. For example, as shown in FIGS. 1, 3, and 4, the sensors can include a thermometer module 108 that can be used to measure body temperature.

In the example shown in FIGS. 1, 3, and 4, the thermometer module 108 is integrated with the medical device 100. The thermometer module 108 includes a port 110 for housing a handheld probe that can be orally inserted into the mouth of a patient to take a temperature reading. Alternatively, the housing 102 of the medical device 100 can be configured to house a handheld probe that can be inserted into an ear of the patient to take a temperature reading.

As shown in FIG. 5, which will be described in more detail further below, the sensors can include a non-invasive blood pressure (NIBP) sensor 106 that includes a cuff that can be inflated around the arm of a patient to non-invasively measure systolic and diastolic blood pressure. The sensors can further include a pulse oximeter sensor 112 that can attach to a finger of the patient to measure vital signs including one or more of blood oxygen saturation (SpO2), pulse rate, and respiration rate. It is contemplated that the medical device 100 can interface with additional types of sensors for measuring additional types of physiological variables.

The sensors can include cables that terminate into connectors that plug into various ports included on the housing 102 of the medical device 100 for transmission of data to the medical device 100. As shown in the example of FIG. 4, the housing 102 includes a first port 150 for connecting the NIBP sensor 106 and a second port 152 for connecting the pulse oximeter sensor 112. The medical device 100 can include a third port 154 for connecting the medical device 100 to a printer. Also, the medical device 100 can include one or more universal serial bus (USB) ports 156, a USB-C port 158, and a registered jack (RJ) interface 160. In some examples, at least some of the sensors wirelessly transmit data to the medical device 100 via a wireless connection established through Wi-Fi, Bluetooth, or another wireless protocol.

In some examples, a scanner can be connected to the medical device 100 such as by plugging into a USB port 156. The scanner can be used to scan machine-readable data such as barcodes, quick-response (QR) codes, and the like that identify a person such as a patient who is being monitored by the medical device 100 or a user of the medical device 100 (e.g., a nurse, a doctor, a caregiver, and the like). The machine-readable data can further identify an object such as medical equipment or a medication being administered to the patient.

The medical device 100 receives data from the sensors for processing. The medical device 100 generates and displays numerical values and/or waveforms based on the data received from the sensors for monitoring the one or more physiological variables of the patient. The medical device 100 displays the numerical values and/or waveforms of the one or more physiological variables on the display 104. Also, the medical device 100 generates alarms when the data received from the sensors indicates an alarm condition is detected.

The alarms generated by the medical device 100 can include a visual alarm displayed on the display 104 and an audible alarm emitted by a speaker of the medical device 100. An additional visual alarm can be displayed on an illumination unit 103, which is positioned on the top surface 111 of the housing 102 of the medical device 100. The illumination unit 103 allows for distant viewing of critical alarms. The critical alarms generated on the illumination unit 103 can be viewed across a 360 degree field of view around the medical device 100. In some examples, both a visual alarm is displayed on the display 104 and a second visual alarm is generated on the illumination unit 103 to indicate a critical status of a detected alarm condition.

The illumination unit 103 can include one or more light-emitting diodes (LEDs) that emit light that is visible through a translucent cover. As an illustrative example, the illumination unit 103 can emit light having a color (e.g., yellow or red) based on a severity of the alarm. Additionally, or alternatively, the illumination unit 103 can emit light flashes based on a predetermined pattern associated with the severity of the alarm (e.g., an increased blinking rate to indicate severe conditions and a decreased blinking rate to indicate less severe conditions).

As further shown in the example of the medical device 100 illustrated in FIGS. 1-4, the housing 102 further includes a plurality of vents 116 for dissipating hot air away from internal components of the medical device. The vents 116 are positioned at different areas of the housing 102 such as on the bottom surface 105 (FIG. 4) and the rear surface 107 (FIG. 2).

FIG. 5 schematically illustrates an example of the medical device 100. In this illustrative example, the medical device 100 includes a computing device 120 having at least one processing device 122 and at least one memory device 124 that stores software instructions that, when executed by the at least one processing device 122, cause the at least one processing device 122 to perform the various aspects, functions, and operations described herein.

The at least one processing device 122 is an example of a processing unit such as a central processing unit (CPU). The at least one processing device 122 can include one or more CPUs. In some examples, the at least one processing device 122 includes one or more digital signal processors, field-programmable gate arrays, or other electronic circuits. The at least one processing device 122 is communicatively coupled to the display 104 and to at least one sensor that captures measurements of one or more physiological variables such as the NIBP sensor 106, the thermometer module 108, and the pulse oximeter sensor 112.

The at least one memory device 124 is an example of a computer-readable data storage device that operates to store data and instructions for execution by the at least one processing device 122. The at least one memory device 124 includes computer-readable media, which includes any media that can be accessed by the at least one processing device 122.

In the example of FIG. 5, the at least one memory device 124 stores a patient baseline tool 126 that when executed by the at least one processing device 122 causes the medical device 100 to adjust one or more alarm limits for one or more physiological variables based on measurements of the one or more physiological variables collected over a predetermined time period. The patient baseline tool 126 will be described in more detail further below.

The computer-readable media can include computer-readable storage media and computer-readable communication media. The computer-readable storage media includes volatile and nonvolatile, removable and non-removable media implemented in any device that stores information such as computer-readable instructions, data structures, program modules, or other data. The computer-readable storage media can include random access memory, read only memory, electrically erasable programmable read only memory, flash memory, and other memory technology, including any medium that can be used to store information for access by the at least one processing device 122. The computer-readable storage media is non-transitory.

The computer-readable communication media embodies computer-readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. The computer-readable communication media can include wired media such as a wired network or direct-wired connection, and wireless media such as acoustic, radio frequency, infrared, and other wireless media. Combinations of any of the above are within the scope of computer-readable media.

As further shown in FIG. 5, the medical device 100 includes a network interface 128 that facilitates connection to a network 20 that can include any type of wired or wireless connections or any combinations thereof. The network interface 128 can include wired interfaces and/or wireless interfaces. For example, the network interface 128 can wirelessly connect the medical device 100 to the network 20 such as through Wi-Fi. Alternatively, or additionally, the network interface 128 can connect the medical device 100 to the network 20 using wired connections such as through Ethernet or Universal Serial Bus (USB) cables.

The network interface 128 enables the medical device 100 to communicate via the network 20 with an electronic medical record (EMR) system 30. The EMR system 30 maintains the medical history of a plurality of patients by consolidating health information for each patient into a single document called an electronic medical record (EMR). Each EMR can include patient demographic information, medical diagnoses, treatment plans, medications, test results, physiological variable measurements, and immunization history. The EMR system 30 enables sharing of data among healthcare professionals to improve coordination of care, reduce errors, and enhance patient outcomes. The EMR system 30 also supports clinical decision-making by providing real-time access to comprehensive patient health information. The EMR system 30 can include secure data storage and integration with other healthcare technologies, which streamline administrative tasks and improve overall healthcare efficiency.

The medical device 100 further includes a sensor interface 130 that operates to communicate with the various sensors that can be communicatively coupled to the medical device 100 such as the NIBP sensor 106, the thermometer module 108, and the pulse oximeter sensor 112. The sensor interface 130 can include both wired interfaces and wireless interfaces. Examples of wireless interfaces can include Wi-Fi, Bluetooth, and other wireless connections. Examples of wired interfaces can include the ports of the medical device 100 (see FIG. 4) that allow connectorized ends of USB or Ethernet cables to plug into the sensor interface 130.

As further shown in FIG. 5, a speaker 132 serves as an integral component of the medical device 100 for providing clear auditory feedback for users and healthcare professionals. The speaker 132 can be used to deliver audible alarms, alerts, notifications, and/or instructions. The speaker 132 ensures that vital information is communicated effectively. Designed for precision and clarity, the speaker 132 helps enhance patient safety by making sure critical data is heard, even in busy or noisy environments which are typical in medical settings.

As shown in FIG. 5, the medical device 100 includes the display 104 which operates to display user interfaces 134. The display 104 can include a touch sensitive screen (e.g., a touchscreen) that receives tactile inputs from the touch of a finger or stylus on its surface. In such examples, the display 104 can include a grid of capacitive or resistive sensors to register locations of touch. In some examples, the display 104 includes a capacitive touchscreen that is coated with a conductive material. When a finger or stylus touches the capacitive touchscreen, the local electrostatic field produced by the conductive material of the display 104 changes, allowing the medical device 100 to determine a point of contact. Alternatively, the display 104 can include a resistive touchscreen that registers pressure applied to the screen by detecting change in resistance when layers of the screen come into contact. The tactile inputs, whether capacitive or resistive, are processed by the computing device 120 to identify actions such as selecting or dragging visual objects that are displayed on the user interfaces 134.

As described above, the medical device 100 can be used to continuously monitor one or more physiological variables of a single patient, or can be used for interval or episodic monitoring of physiological variables of a plurality of patients. During continuous monitoring, the use of default alarms can result in frequent non-actionable alarms that overwhelm users of the medical device 100 such as clinicians, caregivers, and the like. This is especially true for patients who generally ride the borderline of default alarm limits for one or more physiological variables. This can lead to alarm fatigue where users of the medical device 100 begin to consciously and unconsciously ignore the alarms generated by the medical device, which in turn can cause the users of the medical device 100 to miss signs of patient deterioration.

Appropriate patient-specific alarm limits that are based on physiological variable data captured from the patient can be more meaningful and reduce non-actionable alarms. However, users of the medical device 100 are often restricted from changing the default alarm limits to the patient-specific alarm limits without prior authorization because generally there is hesitancy to allow the users of the medical device 100 to change alarm limits for fear that the users could set new alarm limits inappropriately. This means that users of the medical device 100 either do not change the alarm limits to personalize them based on the patient, or that the users request prior authorization to change the alarm limits which can delay the adjustment of the alarm limits. Meanwhile, users may inappropriately silence the alarms on the medical device 100 or become desensitized to the alarms due to alarm fatigue, and thereby miss signs of patient deterioration.

The patient baseline tool 126 addresses the foregoing technical problems by providing a structured presentation of device-generated alarm limit suggestions so that a user can review and decide whether or not to accept the alarm limits. The layout of the patient baseline tool 126 facilitates identification, analysis, and enablement of device-suggested alarm limits. Further, the patient baseline tool 126 facilitates personalization of the alarm limits used by the medical device 100 while still restricting manual change of alarm limits by users.

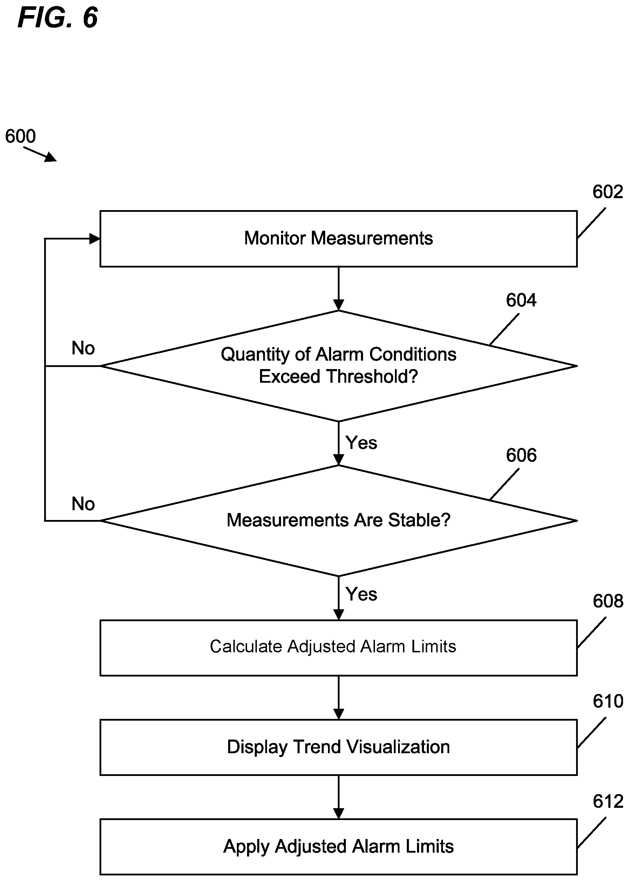

FIG. 6 schematically illustrates an example of a method 600 of monitoring one or more physiological variables of a subject such as a patient admitted to a healthcare facility. The method 600 is performed by the patient baseline tool 126 executed on the medical device 100.

The method 600 includes an operation 602 of monitoring measurements of the one or more physiological variables over a predetermined time period to detect alarm conditions that occur when the measurements of the one or more physiological variables exceed a default upper alarm limit or are less than a default lower alarm limit. Operation 602 can include receiving data from one or more sensors communicatively coupled to the medical device 100 such as the NIBP sensor 106, the thermometer module 108, and the pulse oximeter sensor 112. Operation 602 can further include determining measurement values of the one or more physiological variables based on the data received from the sensors communicatively coupled to the medical device 100.

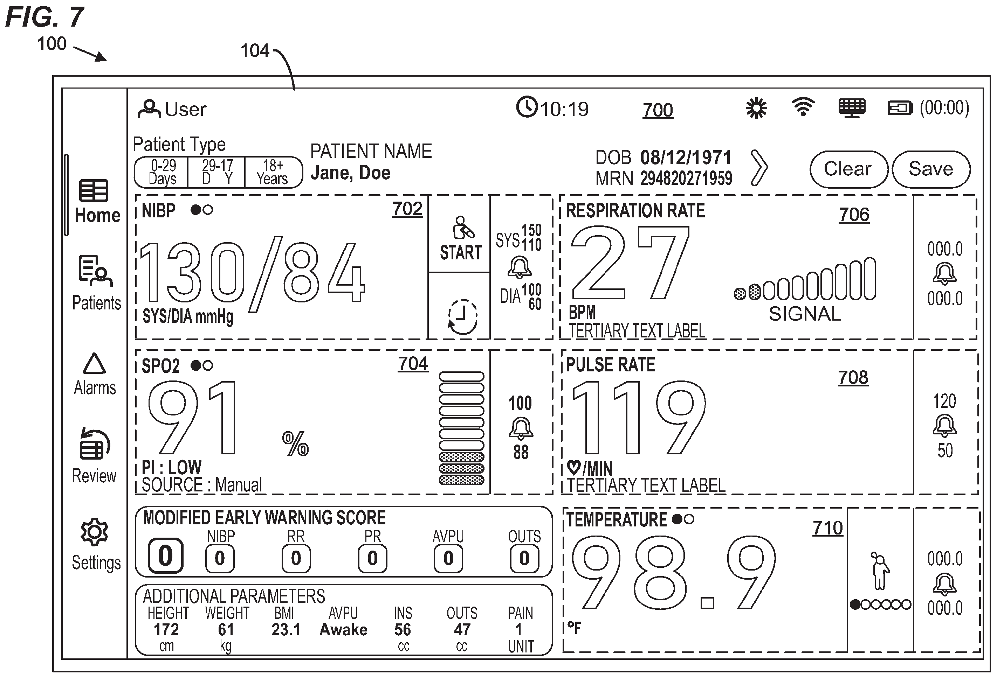

FIG. 7 illustrates an example of a home screen user interface 700 that can be displayed on the display 104 of the medical device 100 to display the measurements of the one or more physiological variables monitored in operation 602 of the method 600. The home screen user interface 700 includes a non-invasive blood pressure (NIBP) display area 702 that displays systolic and diastolic blood pressure measurements based on the data received from the NIBP sensor 106. The home screen user interface 700 further includes a blood oxygen saturation (SpO2) display area 704 that displays SpO2 measurements, a respiration rate display area 706 that displays respiration rate measurements, and a pulse rate display area 708 that displays pulse rate measurements based on the data received from the pulse oximeter sensor 112. The home screen user interface 700 further includes a temperature display area 710 that displays temperature measurements based on data from the thermometer module 108. In this example, none of the NIBP display area 702, SpO2 display area 704, respiration rate display area 706, pulse rate display area 708, and temperature display area 710 show that an alarm condition is present for any of the physiological variables monitored by the medical device 100.

The home screen user interface 700 can display additional data such as the name, date of birth, and medical record number (MRN) of the patient who is being monitored by the medical device 100. The home screen user interface 700 can also display health scores such as a modified early warning score, and additional patient data such as height, weight, body mass index (BMI), Alert, Verbal, Pain, and Unresponsive (AVPU) scale, pain level, and the like.

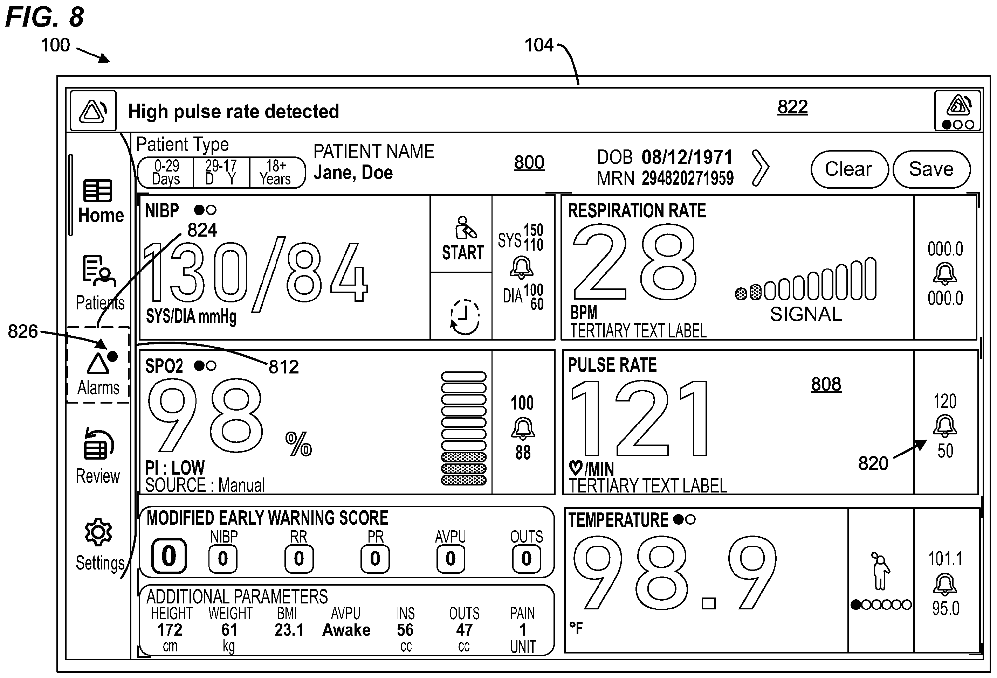

FIG. 8 illustrates an example of a home screen user interface 800 when an alarm condition is detected by the medical device 100 such as during operation 602 of the method 600. In this illustrative example, an alarm condition is detected based on the pulse rate measurements displayed in the pulse rate display area 808. For example, the pulse rate display area 808 displays a pulse rate measurement of 121 beats per minute (bpm) which exceeds a default upper alarm limit of 120 bpm. In this example, a default lower alarm limit of 50 bpm is set for the pulse rate.

As shown in FIG. 8, when the alarm condition is detected by the medical device 100, a border of the display area with the detected alarm condition, in this case the pulse rate display area 808, is illuminated (e.g., in the color red) to distinguish the display area from the other display areas of the home screen user interface 800. Also, an alarm icon 820 is illuminated (e.g., in the color red) inside the display area with the alarm condition. Further, an alarm ribbon 822 is displayed across the home screen user interface 800 such as across a top portion of the interface. The alarm ribbon 822 identifies the alarm condition (e.g., "High pulse rate detected"). Both the alarm icon 820 and the alarm ribbon 822 are selectable by touching the display 104 with a finger or stylus such as to silence or pause the alarm triggered on the medical device 100.

Referring back to FIG. 6, the method 600 includes an operation 604 of determining whether a quantity of the alarm conditions detected during the predetermined time period exceeds a threshold set for the predetermined time period. The quantity of the alarm conditions detected during the predetermined time period is compared to the threshold to determine whether the alarms are frequent non-actionable alarms (also known as nuisance alarms) which typically occurs for patients who ride the borderline of default alarm limits for one or more physiological variables. As an illustrative example, athletes typically have a lower resting pulse rate than persons who are not athletes such that when being continuously monitored by the medical device 100, the pulse rate of an athlete can ride the borderline of the default lower alarm limit set for the pulse rate which can cause the triggering of frequent non-actionable alarms.

As an illustrative example, the predetermined time period is 30 minutes, 60 minutes, or 120 minutes depending on the type of physiological variable. In the case of pulse rate, the predetermined time period can be set for 60 minutes. The threshold can be set for a quantity equal to or greater than a number of alarms triggered within the predetermined time period. The threshold may differ for different physiological variables and different predetermined time periods. In the case of pulse rate, the threshold can be set for a quantity equal to or greater than five alarms triggered within the predetermined time period of 60 minutes.

Using pulse rate as an illustrative example, when an alarm is triggered five or more times within the 60 minute predetermined time period, operation 604 determines that the quantity of the alarms exceeds the threshold (i.e., "Yes" in operation 604) and the method 600 proceeds to an operation 606 of determining whether the measurements of the physiological variable are stable within the predetermined time period. Otherwise, when the quantity of the alarms does not exceed the threshold (i.e., "No" in operation 604), the method 600 returns to operation 602 to continue monitoring the measurements of the one or more physiological variables.

Operation 606 is performed to confirm that the physiological variable measurements are not due to health deterioration but are instead typical for the patient, such as when the measurements of the physiological variable ride a borderline of a default upper alarm limit or a borderline of a default lower alarm limit which causes frequent non-actionable alarms.

As an illustrative example, operation 606 can include determining whether the measurements of the physiological variable are stable or not by comparing a coefficient of variation of the measurements within the predetermined time period to a threshold. For example, when the measurements of the physiological variable within the predetermined time period have a coefficient of variation that is less than 0.1, the measurements are determined to be stable. When the measurements of the physiological variable within the predetermined time period have a coefficient of variation that is equal to or greater than 0.1, the measurements are determined to be unstable (i.e., "No" in operation 606) such that the method 600 returns to operation 602 to continue monitoring the measurements of the one or more physiological variables.

It is contemplated that operation 606 can include alternative metrics of measuring stability in addition to the example of using coefficient of variation. For example, operation 606 can include calculating a standard deviation, a standard error, a mean absolute deviation, or other metric that can be compared to a threshold value to determine whether the measurements of the physiological variable are stable within the predetermined time period.

In some instances, measurements based on the data captured by the sensors communicatively coupled to the medical device 100 can be disrupted or ignored due to noise such as from movement of the patient, or by other environmental conditions. In such instances, the medical device 100 receives no measurements or a sparse quantity of measurements within the predetermined time period such that determining whether the measurements of the physiological variable are stable can be unreliable due to the lack of sufficient data for analysis.

Accordingly, in some examples, operation 606 can include determining whether there is a sufficient quantity of measurements captured within the predetermined time period to ensure the accuracy of determining whether the measurements are stable. For example, the quantity of measurements captured within the predetermined time period can be compared to a minimum quantity threshold to determine whether there is a sufficient quantity of measurements captured within the predetermined time period. When the quantity of measurements captured within the predetermined time period is less than the minimum quantity threshold, operation 606 cannot determine whether the measurements are stable. In such instances, the measurements are considered unstable (i.e., "No" in operation 606) such that the method 600 returns to operation 602 to continue monitoring the measurements of the one or more physiological variables.

When the measurements of the physiological variable are determined to be stable within the predetermined time period (i.e., "Yes" in operation 606), the method 600 proceeds to an operation 608 of calculating adjusted alarm limits based on the physiological variable measurements within the predetermined time period. The adjusted alarm limits can include at least one of an adjusted upper alarm limit that differs from the default upper alarm limit and an adjusted lower alarm limit that differs from the default lower alarm limit.

As an illustrative example, the adjusted alarm limits are calculated by increasing at least one of the default upper alarm limit and the default lower alarm limit based on a standard deviation of the measurements of the physiological variable over the predetermined time period. Alternatively, the adjusted alarm limits can be calculated by decreasing at least one of the default upper alarm limit and the default lower alarm limit based on the standard deviation of the measurements of the physiological variable over the predetermined time period. For example, the default upper and lower alarm limits can shift by one, two, or more standard deviations, or can shift by less than one standard deviation such as by half a standard deviation. It is contemplated that alternative metrics can be used to determine the adjusted alarm limits in operation 608.

When the adjusted alarm limits are calculated by the patient baseline tool 126 such that they are ready for review by a user of the medical device 100, the home screen user interface 800 (see FIG. 8) is updated to display an indicator 826 in an alarm tab 824 of a menu 812. The indicator 826 can be a dot that is illuminated inside the alarm tab 824.

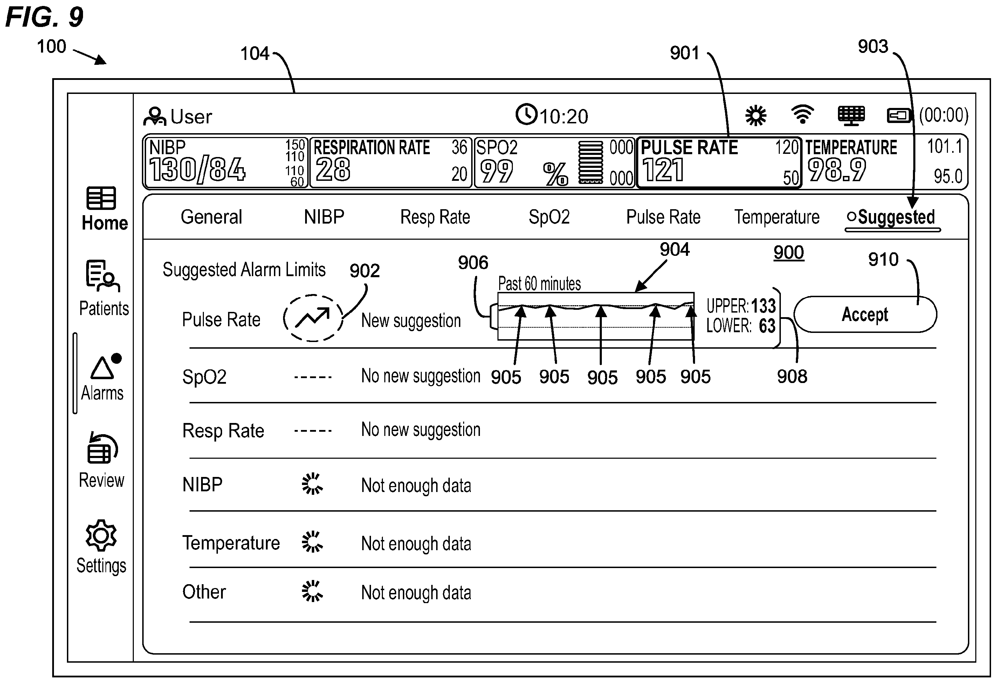

The method 600 includes an operation 610 of displaying, via a graphical user interface on the display 104, a trend visualization showing the measurements of the physiological variable over the predetermined time period relative to the default alarm limits and the adjusted alarm limits. An illustrative example of the trend visualization is shown in FIG. 9.

FIG. 9 illustrates an example of an alarm tab user interface 900 that is displayed on the display 104 of the medical device 100 in response to selection of the alarm tab 824 in the menu 812 of the home screen user interface 800 of FIG. 8. In the example of FIG. 9, a border of a pulse rate measurement display area 901 is illuminated (e.g., in the color red) to visually indicate that the pulse rate measurements are causing the medical device 100 to generate an alarm. Further, the alarm tab user interface 900 is displayed as a result of a selection of a suggested alarm tab 903 on the display 104. The alarm tab user interface 900 enables a user of the medical device 100 to review suggested alarm limits generated by the patient baseline tool 126 for monitoring a physiological variable while also viewing a graphical representation of a trend of the physiological variable over the predetermined time period.

In addition to or as an alternative to the alarm tab user interface 900, the suggested alarm limits calculated by the patient baseline tool 126 can be displayed on a home screen user interface (see examples shown in FIGS. 7 and 8), in a pop-up window, or other type of screen or window that can be displayed on the display 104 of the medical device 100.

In this illustrative example, the alarm tab user interface 900 displays a suggestion symbol 902 that indicates that there is suggested modification of the default alarm limits used for monitoring pulse rate. The alarm tab user interface 900 further displays a trend visualization 904 of measurements for a physiological variable (i.e., pulse rate) over a predetermined time period (e.g., 60 minutes). Default alarm limits 906, which include a default upper alarm limit and a default lower alarm limit, are shown relative to the trend visualization 904. In this illustrative example, the trend visualization 904 shows that the pulse rate measurements ride along the default upper alarm limit such that an alarm condition 905 was triggered five times in the past 60 minutes which exceeds the threshold in operation 604 of the method 600.

The alarm tab user interface 900 further displays values for adjusted alarm limits 908. For example, the adjusted alarm limits 908 include a value for an adjusted upper alarm limit and a value of an adjusted lower alarm limit. In this illustrative example, the adjusted upper alarm limit has a value of 133 BPM which represents an increase of three standard deviations (3σ) (e.g., 13 bpm) away from the default upper alarm limit of 120 BPM. Similarly, the adjusted lower alarm limit has a value of 63 BPM which represents an increase of three standard deviations (3σ) (e.g., 13 bpm) away from the default lower alarm limit of 50 BPM.

In the example shown in FIG. 9, the patient baseline tool 126 is operating under an active mode in which the adjusted alarm limits 908 are selectable for monitoring the pulse rate. Accordingly, in this illustrative example, the alarm tab user interface 900 includes an accept icon 910 that when selected on the display 104 causes the pulse rate to be monitored using the adjusted alarm limits 908 having the adjusted upper and lower alarm limit values.

Accordingly, the alarm tab user interface 900 allows the user of the medical device 100 to decide whether they want to accept the suggested alarm limits, or not. When the user of the medical device 100 does not select the accept icon 910 to accept the suggested alarm limits, the default alarm limits 906 are used for monitoring the physiological variable.

In examples where the patient baseline tool 126 operates under the active mode, the method 600 can include an operation 612 of applying the adjusted alarm limits when the accept icon 910 is selected on the display 104 of the medical device 100. Advantageously, this will decrease the frequency of non-actionable alarms generated by the medical device 100 because the pulse rate is less likely to exceed the adjusted upper alarm limit because the adjusted upper alarm limit takes into consideration that the patient has an abnormally high pulse rate, as exhibited by the patient over the predetermined time period shown in the trend visualization 904.

In some examples, the patient baseline tool 126 prevents a user of the medical device 100 from adjusting the default alarm limits besides acceptance of the adjusted alarm limits 908 that are calculated by the patient baseline tool 126 in operation 608. Accordingly, in such examples, the patient baseline tool 126 is the only way to adjust alarm limits on the medical device 100. This would enable users of the medical device 100 to change the alarm limits used by the medical device 100 but only within confines prescribed by a facility where the medical device 100 is being used such as a hospital, medical clinic, or other healthcare environment.

Alternatively, the patient baseline tool 126 can operate under a demo mode in which the adjusted alarm limits 908 are not selectable for monitoring the alarm conditions of the physiological variable. In such examples, the alarm tab user interface 900 does not display the accept icon 910, or displays the accept icon 910 as greyed out to visually indicate that it is unavailable or inactive such that the accept icon 910 cannot be selected on the display 104.

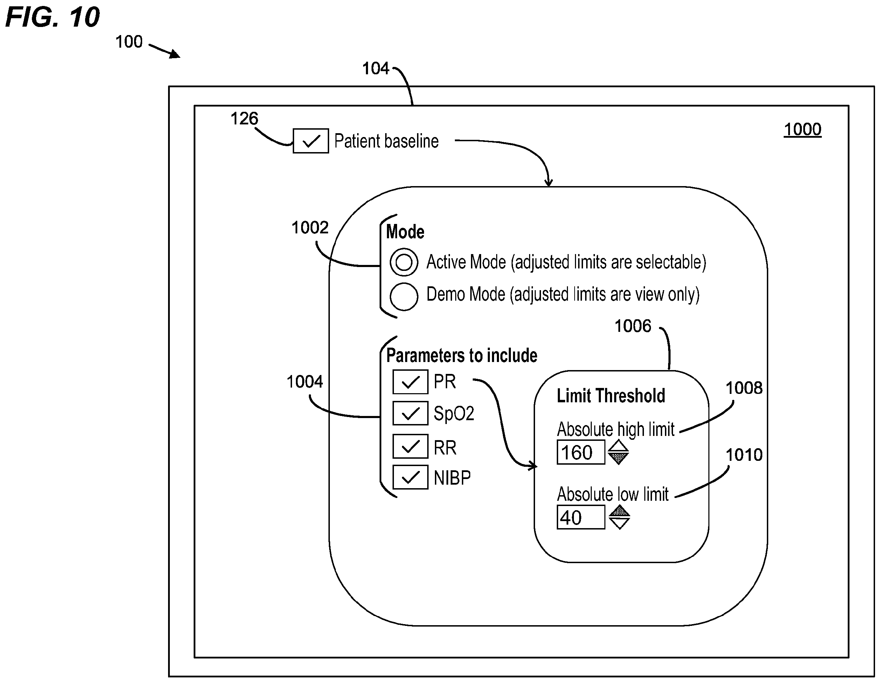

FIG. 10 illustrates an example of a configuration user interface 1000 that can be displayed on the display 104 of the medical device 100 to configure the patient baseline tool 126. In this example, the configuration user interface 1000 includes a first set of inputs 1002 for selection of the active mode in which the adjusted alarm limits are selectable for monitoring the alarm conditions or selection of the demo mode in which the adjusted alarm limits are not selectable for monitoring the alarm conditions. Accordingly, when the patient baseline tool 126 is selected for execution on the medical device 100, the patient baseline tool 126 can be activated immediately (i.e., operate under the active mode) such that the suggested alarm limits are selectable for use, or the patient baseline tool 126 can operate in the demo mode such that the suggested alarm limits are view only. The demo mode can be advantageous when users of the medical device 100 want to become accustomed to the suggested alarm limits and/or want to build trust around the patient baseline tool 126 before deploying the tool.

In the example shown in FIG. 10, the first set of inputs 1002 include graphical user interface elements (e.g., small circles) that allow users to select one option from a group of mutually exclusive options. When a graphical user interface element is selected, a dot or marker appears inside the element to indicate the selection. Clicking on an element selects it and deselects the other element within the first set of inputs 1002.

The configuration user interface 1000 further includes a second set of inputs 1004 for selection of one or more physiological variables for monitoring with the patient baseline tool. The second set of inputs 1004 include graphical user interface elements (e.g., empty squares) that when selected cause a marker such as a checkmark to appear inside the element to indicate the selection. Clicking on an element twice deselects the element such that the mark disappears. In the illustrated example, the second set of inputs 1004 includes elements for selecting one or more of pulse rate, SpO2, respiration rate, and non-invasive blood pressure (NIBP).

As shown in FIG. 10, for each physiological variable selected using the second set of inputs 1004, the configuration user interface 1000 displays a limit threshold window 1006 that allows selection of a maximum limit 1008 for the adjusted upper alarm limit and a minimum limit 1010 for the adjusted lower alarm limit. The adjusted upper alarm limit cannot exceed the maximum limit 1008 selected in the limit threshold window 1006 for the physiological variable and the adjusted lower alarm limit cannot be less than the minimum limit 1010 selected in the limit threshold window 1006 for the physiological variable.

In the example illustrated in FIG. 10, the maximum limit 1008 is set to 160 bpm for the pulse rate which can be numerically adjusted using up and down arrows. The minimum limit 1010 is set to 40 bpm for the pulse rate which can also be numerically adjusted using up and down arrows. The maximum and minimum limits can be adjusted/selected for each physiological variable selected using the second set of inputs 1004 of the configuration user interface 1000.

In some examples, the maximum limits 1008 for the adjusted upper alarm limits cannot exceed absolute upper limits set by a manufacturer of the medical device 100. Similarly, the minimum limits 1010 for the adjusted lower alarm limits cannot be lower than absolute lower limits set by the manufacturer of the medical device 100. Accordingly, the configuration user interface 1000 allows a user to set maximum and minimum limits that are tighter than the absolute maximum and minimum limits set by the manufacturer of the medical device 100 but that can never go beyond the absolute limits to ensure patient safety.

The medical device 100 provides a technological solution to the specific technical problem of alarm fatigue in medical monitoring systems. As described above, nuisance alarms can lead to healthcare professionals overlooking or ignoring alarms, which jeopardizes patient safety. The medical device 100 implements a specific improvement to alarm management by using a structured, rules-based approach to automatically analyze alarm patterns and suggest optimized alarm limits for one or more physiological variables monitored by the device.

The technical solution includes monitoring physiological measurements over a predetermined time period using default alarm limits to detect alarm pattern frequency, analyzing measurement stability using coefficient of variation calculations, and automatically generating adjusted alarm limits based on statistical analysis of the measurement data. This represents a solution that is necessarily rooted in medical monitoring technology.

Further, the patient baseline tool 126 improves the functioning of medical device 100 by reducing non-actionable alarms while maintaining patient safety through a structured review process. The patient baseline tool 126 provides an interactive graphical user interface that displays trend visualizations showing physiological measurements relative to both default and adjusted alarm limits, enabling users to make informed decisions about alarm limit adjustments. This represents a specific technical implementation on the medical device 100.

Additionally, the technical implementation includes specific hardware components working together, including sensors for capturing physiological measurements, a display, and memory storing specialized software instructions for execution by a processing device on the medical device 100. The patient baseline tool 126 provides a specific way to solve the technological problem of alarm fatigue through an ordered combination of specific rules and thresholds applied to sensor data, statistical analysis of measurement stability, and a structured user interface for reviewing suggested alarm limit adjustments.

The patient baseline tool 126 improves upon conventional medical device alarm systems by providing automated analysis of alarm patterns and measurement stability to suggest optimized alarm limits, while maintaining patient safety through maximum and minimum threshold constraints that cannot exceed manufacturer-set absolute limits. This represents a technological solution that requires complex analysis of sensor data streams, statistical calculations, and real-time display of trend visualizations.

The specific technical implementation is illustrated in the figures showing hardware components of the medical device (FIGS. 1-4), system architecture (FIG. 5), processing flow (FIG. 6), and specialized user interfaces (FIGS. 7-10) that enable improved alarm management functionality. These technical details demonstrate that the medical device 100 provides a specific technological solution to a technological problem in medical monitoring systems.

Embodiments of the present disclosure can be described with reference to the following numbered clauses, with preferred features laid out in the dependent clauses:
1. A medical device for monitoring one or more physiological variables, the medical device comprising:
   at least one processing device communicatively coupled to a display and to at least one sensor that captures measurements of the physiological variable; and
   a memory device storing instructions which, when executed by the at least one processing device, cause the at least one processing device to:
      monitor the measurements of the physiological variable over a predetermined time period to detect alarm conditions when the measurements of the physiological variable exceed a default upper alarm limit or are less than a default lower alarm limit;
      determine whether a quantity of the alarm conditions detected during the predetermined time period exceeds a threshold set for the predetermined time period;
      when the quantity of the alarm conditions exceeds the threshold set for the predetermined time period, determine whether the measurements of the physiological variable are stable within the predetermined time period;
      when the measurements of the physiological variable are stable within the predetermined time period, execute a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and
      display, via a graphical user interface on the display, a trend visualization showing the measurements of the physiological variable relative to the default alarm limits and the adjusted alarm limits.
2. The medical device of clause 1, wherein the memory device stores additional instructions which, when executed by the at least one processing device, further cause the at least one processing device to:
   operate the patient baseline tool under a demo mode in which the adjusted alarm limits are not selectable for monitoring the alarm conditions of the physiological variable.
3. The medical device of any one of clauses 1-2, wherein the memory device stores additional instructions which, when executed by the at least one processing device, further cause the at least one processing device to:
   operate the patient baseline tool under an active mode in which the adjusted alarm limits are selectable for monitoring the alarm conditions of the physiological variable.
4. The medical device of any one of clauses 1-3, wherein the predetermined time period is 30 minutes, 60 minutes, or 120 minutes.
5. The medical device of any one of clauses 1-4, wherein the measurements of the physiological variable are stable when a coefficient of variation of the measurements is less than a threshold.
6. The medical device of any one of clauses 1-5, wherein the adjusted alarm limits are calculated by increasing at least one of the default upper alarm limit and the default lower alarm limit by one or more standard deviations of the measurements of the physiological variable calculated over the predetermined time period.
7. The medical device of any one of clauses 1-6, wherein the adjusted alarm limits are calculated by decreasing at least one of the default upper alarm limit and the default lower alarm limit by one or more standard deviations of the measurements of the physiological variable calculated over the predetermined time period.
8. The medical device of any one of clauses 1-7, wherein the memory device stores additional instructions which, when executed by the at least one processing device, further cause the at least one processing device to:
   display a configuration interface to configure the patient baseline tool for execution on the medical device, the configuration interface enabling:
   selection of an active mode in which the adjusted alarm limits are selectable for monitoring the alarm conditions or a demo mode in which the adjusted alarm limits are not selectable for monitoring the alarm conditions;
   selection of one or more physiological variables for the patient baseline tool; and
   for each physiological variable selected for the patient baseline tool, selection of a maximum threshold for the adjusted upper alarm limit and a minimum threshold for the adjusted lower alarm limit.
9. The medical device of clause 8, wherein the maximum thresholds for the adjusted upper alarm limits cannot exceed absolute upper limit thresholds set by a manufacturer of the medical device, and the minimum thresholds for the adjusted lower alarm limits cannot be lower than absolute lower limit thresholds set by the manufacturer of the medical device.
10. A method of monitoring one or more physiological variables, the method comprising:
   monitoring measurements of a physiological variable over a predetermined time period to detect alarm conditions when the measurements of the physiological variable exceed a default upper alarm limit or are less than a default lower alarm limit;
   determining whether a quantity of the alarm conditions detected during the predetermined time period exceeds a threshold set for the predetermined time period;
   when the quantity of the alarm conditions exceeds the threshold set for the predetermined time period, determining whether the measurements of the physiological variable are stable within the predetermined time period;
   when the measurements of the physiological variable are stable within the predetermined time period, executing a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and
   displaying a trend visualization showing the measurements of the physiological variable relative to the default alarm limits and the adjusted alarm limits.
11. The method of clause 10, further comprising:
   operating the patient baseline tool under a demo mode in which the adjusted alarm limits are not selectable for monitoring the alarm conditions of the physiological variable.
12. The method of any one of clauses 10-11, further comprising:
   operating the patient baseline tool under an active mode in which the adjusted alarm limits are selectable for monitoring the alarm conditions of the physiological variable.
13. The method of any one of clauses 10-12, wherein the predetermined time period is 30 minutes, 60 minutes, or 120 minutes.
14. The method of any one of clauses 10-13, wherein the measurements of the physiological variable are stable when a coefficient of variation of the measurements is less than a threshold.
15. The method of any one of clauses 10-14, wherein the adjusted alarm limits are calculated by increasing at least one of the default upper alarm limit and the default lower alarm limit by one or more standard deviations of the measurements of the physiological variable calculated over the predetermined time period.
16. The method of any one of clauses 10-15, wherein the adjusted alarm limits are calculated by decreasing at least one of the default upper alarm limit and the default lower alarm limit by one or more standard deviations of the measurements of the physiological variable calculated over the predetermined time period.
17. The method of any one of clauses 10-16, further comprising:
   displaying a configuration interface to configure the patient baseline tool for execution on the medical device, the configuration interface enabling:
   selection of an active mode in which the adjusted alarm limits are selectable for monitoring the alarm conditions or a demo mode in which the adjusted alarm limits are not selectable for monitoring the alarm conditions;
   selection of one or more physiological variables for the patient baseline tool; and
   for each physiological variable selected for the patient baseline tool, selection of a maximum threshold for the adjusted upper alarm limit and a minimum threshold for the adjusted lower alarm limit.
18. The method of any one of clauses 10-17, wherein the maximum thresholds for the adjusted upper alarm limits cannot exceed absolute upper limit thresholds set by a manufacturer of the medical device, and the minimum thresholds for the adjusted lower alarm limits cannot be lower than absolute lower limit thresholds set by the manufacturer of the medical device.
19. The method of any one of clauses 10-18, wherein the one or more physiological variables include pulse rate, blood oxygen saturation (SpO2), respiration rate, and non-invasive blood pressure.
20. A computer program product storing instructions that, when executed by at least one processing device communicatively coupled to a display and to at least one sensor that captures measurements of a physiological variable, cause the at least one processing device to:
   monitor the measurements of the physiological variable over a predetermined time period to detect alarm conditions when the measurements of the physiological variable exceed a default upper alarm limit or are less than a default lower alarm limit;
   determine whether a quantity of the alarm conditions detected during the predetermined time period exceeds a threshold set for the predetermined time period;
   when the quantity of the alarm conditions exceeds the threshold set for the predetermined time period, determine whether the measurements of the physiological variable are stable within the predetermined time period;
   when the measurements of the physiological variable are stable within the predetermined time period, execute a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and
   display, via a graphical user interface on the display, a trend visualization showing the measurements of the physiological variable relative to the default alarm limits and the adjusted alarm limits.

## Claims

1. A medical device for monitoring one or more physiological variables, the medical device comprising:
at least one processing device communicatively coupled to a display and to at least one sensor that captures measurements of the physiological variable; and
a memory device storing instructions which, when executed by the at least one processing device, cause the at least one processing device to:
monitor the measurements of the physiological variable over a predetermined time period to detect alarm conditions when the measurements of the physiological variable exceed a default upper alarm limit or are less than a default lower alarm limit;
determine whether a quantity of the alarm conditions detected during the predetermined time period exceeds a threshold set for the predetermined time period;
when the quantity of the alarm conditions exceeds the threshold set for the predetermined time period, determine whether the measurements of the physiological variable are stable within the predetermined time period;
when the measurements of the physiological variable are stable within the predetermined time period, execute a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and
display, via a graphical user interface on the display, a trend visualization showing the measurements of the physiological variable relative to the default alarm limits and the adjusted alarm limits.

2. The medical device of claim 1, wherein the memory device stores additional instructions which, when executed by the at least one processing device, further cause the at least one processing device to:
operate the patient baseline tool under a demo mode in which the adjusted alarm limits are not selectable for monitoring the alarm conditions of the physiological variable.

3. The medical device of any one of claims 1-2, wherein the memory device stores additional instructions which, when executed by the at least one processing device, further cause the at least one processing device to:
operate the patient baseline tool under an active mode in which the adjusted alarm limits are selectable for monitoring the alarm conditions of the physiological variable.

4. The medical device of any one of claims 1-3, wherein the predetermined time period is 30 minutes, 60 minutes, or 120 minutes.

5. The medical device of any one of claims 1-4, wherein the measurements of the physiological variable are stable when a coefficient of variation of the measurements is less than a threshold.

6. The medical device of any one of claims 1-5, wherein the adjusted alarm limits are calculated by increasing at least one of the default upper alarm limit and the default lower alarm limit by one or more standard deviations of the measurements of the physiological variable calculated over the predetermined time period.

7. The medical device of any one of claims 1-6, wherein the adjusted alarm limits are calculated by decreasing at least one of the default upper alarm limit and the default lower alarm limit by one or more standard deviations of the measurements of the physiological variable calculated over the predetermined time period.

8. The medical device of any one of claims 1-7, wherein the memory device stores additional instructions which, when executed by the at least one processing device, further cause the at least one processing device to:
display a configuration interface to configure the patient baseline tool for execution on the medical device, the configuration interface enabling:
selection of an active mode in which the adjusted alarm limits are selectable for monitoring the alarm conditions or a demo mode in which the adjusted alarm limits are not selectable for monitoring the alarm conditions;
selection of one or more physiological variables for the patient baseline tool; and
for each physiological variable selected for the patient baseline tool, selection of a maximum threshold for the adjusted upper alarm limit and a minimum threshold for the adjusted lower alarm limit.

9. The medical device of claim 8, wherein the maximum thresholds for the adjusted upper alarm limits cannot exceed absolute upper limit thresholds set by a manufacturer of the medical device, and the minimum thresholds for the adjusted lower alarm limits cannot be lower than absolute lower limit thresholds set by the manufacturer of the medical device.

10. A method of monitoring one or more physiological variables, the method comprising:
monitoring measurements of a physiological variable over a predetermined time period to detect alarm conditions when the measurements of the physiological variable exceed a default upper alarm limit or are less than a default lower alarm limit;
determining whether a quantity of the alarm conditions detected during the predetermined time period exceeds a threshold set for the predetermined time period;
when the quantity of the alarm conditions exceeds the threshold set for the predetermined time period, determining whether the measurements of the physiological variable are stable within the predetermined time period;
when the measurements of the physiological variable are stable within the predetermined time period, executing a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and
displaying a trend visualization showing the measurements of the physiological variable relative to the default alarm limits and the adjusted alarm limits.

11. The method of claim 10, wherein the measurements of the physiological variable are stable when a coefficient of variation of the measurements is less than a threshold.

12. The method of any one of claims 10-11, wherein the adjusted alarm limits are calculated by increasing at least one of the default upper alarm limit and the default lower alarm limit by one or more standard deviations of the measurements of the physiological variable calculated over the predetermined time period.

13. The method of any one of claims 10-12, further comprising:
displaying a configuration interface to configure the patient baseline tool for execution on the medical device, the configuration interface enabling:
selection of an active mode in which the adjusted alarm limits are selectable for monitoring the alarm conditions or a demo mode in which the adjusted alarm limits are not selectable for monitoring the alarm conditions;
selection of one or more physiological variables for the patient baseline tool; and
for each physiological variable selected for the patient baseline tool, selection of a maximum threshold for the adjusted upper alarm limit and a minimum threshold for the adjusted lower alarm limit.

14. The method of any one of claims 10-13, wherein the maximum thresholds for the adjusted upper alarm limits cannot exceed absolute upper limit thresholds set by a manufacturer of the medical device, and the minimum thresholds for the adjusted lower alarm limits cannot be lower than absolute lower limit thresholds set by the manufacturer of the medical device.

15. A computer program product storing instructions that, when executed by at least one processing device communicatively coupled to a display and to at least one sensor that captures measurements of a physiological variable, cause the at least one processing device to:
monitor the measurements of the physiological variable over a predetermined time period to detect alarm conditions when the measurements of the physiological variable exceed a default upper alarm limit or are less than a default lower alarm limit;
determine whether a quantity of the alarm conditions detected during the predetermined time period exceeds a threshold set for the predetermined time period;
when the quantity of the alarm conditions exceeds the threshold set for the predetermined time period, determine whether the measurements of the physiological variable are stable within the predetermined time period;
when the measurements of the physiological variable are stable within the predetermined time period, execute a patient baseline tool that calculates adjusted alarm limits that include at least one of an adjusted upper alarm limit and an adjusted lower alarm limit, the adjusted upper alarm limit differing from the default upper alarm limit and the adjusted lower alarm limit differing from the default lower alarm limit; and
display, via a graphical user interface on the display, a trend visualization showing the measurements of the physiological variable relative to the default alarm limits and the adjusted alarm limits.
